## Europäisches Patentamt

### European Patent Office

### Office européen des brevets

(11) Veröffentlichungsnummer: **0 043 941**
**B1**

(12) **EUROPÄISCHE PATENTSCHRIFT**

(45) Veröffentlichungstag der Patentschrift:
**29.05.85**

(21) Anmeldenummer: **81104790.1**

(22) Anmeldetag: **22.06.81**

(51) Int. Cl.⁴: **G 01 N 33/18**, G 01 N 27/06

(54) Verfahren zur Überwachung von Reinstwasser auf Fremdionengehalt mit Hilfe einer Leitfähigkeitsmessung.

(30) Priorität: **03.07.80 DE 3025275**

(43) Veröffentlichungstag der Anmeldung:
**20.01.82 Patentblatt 82/3**

(45) Bekanntmachung des Hinweises auf die Patenterteilung:
**29.05.85 Patentblatt 85/22**

(84) Benannte Vertragsstaaten:
**CH DE GB LI NL**

(56) Entgegenhaltungen:
**DE - C - 942 418**
**FR - A - 2 288 310**
**US - A - 4 190 827**

(73) Patentinhaber: **Siemens Aktiengesellschaft, Berlin und München Wittelsbacherplatz 2, D-8000 München 2 (DE)**

(72) Erfinder: **Maurer, Otto, Dr., Bussardweg 6, D-6729 Wörth (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

## Beschreibung

Die Erfindung bezieht sich auf ein Verfahren zur Überwachung von Reinstwasser auf Fremdionengehalt mit Hilfe einer Leitfähigkeitsmessung.

Viele industrielle Prozesse verlangen die Verwendung von Reinstwasser, welches durch Aufbereitung aus Brauchwasser mit relativ hohem Elektrolytgehalt gewonnen wird, z. B. mit Hilfe von Ionenaustauschern.

Zur kontinuierlichen Überwachung des Elektrolytgehalts in Reinstwasser wird bevorzugt die Leitfähigkeitsmessung herangezogen. Üblicherweise wird die Messung bei Umgebungstemperatur oder höheren Temperaturen vorgenommen und das Meßsignal auf eine Temperatur von 20 bzw. 25° C kompensiert.

Bedingt durch die Eigendissoziation des Wassers ergibt sich bei den sehr geringen Fremdionengehalten, denen die Überwachung gilt, ein schlechtes Nutzsignal-/Störsignal-Verhältnis, so daß das gewonnene Meßsignal keine eindeutige Aussage über den Fremdionengehalt des überwachten Wassers zuläßt.

Es besteht somit die Aufgabe, die Meßempfindlichkeit und damit das Nutzsignal-/Störsignal-Verhältnis so zu verbessern, daß auch sehr geringe Fremdionenkonzentrationen sicher erkannt und ihre Änderung über die Zeit durch Aufzeichnung des Meßsignals verfolgt werden kann.

Eine Lösung der Aufgabe wird in einem Verfahren der eingangs genannten Art gesehen, das dadurch gekennzeichnet ist, daß das Wasser vor der Messung auf eine mindestens annähernd konstante Temperatur zwischen 0° C und 5° C gekühlt wird.

Es hat sich als vorteilhaft herausgestellt, das zu untersuchende Wasser auf eine Temperatur in unmittelbarer Nähe seines Gefrierpunkts zu kühlen.

Es kann dazu ein Thermostat mit exakter Temperaturregelung verwendet werden, oder es wird eine gewisse Schwankungsbreite der Temperaturregelung zugelassen und das in der Leitfähigkeitsmeßeinrichtung gewonnene Meßsignal in diesem kleinen Bereich in üblicher Weise temperaturkompensiert.

Mit Hilfe dieses wenig aufwendigen Verfahrens wird es möglich, die durch Säuren, Laugen oder Salze hervorgerufenen elektrolytischen Verunreinigungen kleinster Konzentration im Reinstwasser zu erkennen und frühzeitig entsprechende Maßnahmen zu treffen.

Bei basischen Verunreinigungen tritt eine eigenartige Extremwertbildung der Empfindlichkeitskennlinie im Bereich niedrigster Kationenkonzentrationen auf, in deren Bereich mit Sicherheit auf Reinstwasser mit leicht alkalischer Reaktion geschlossen werden kann.

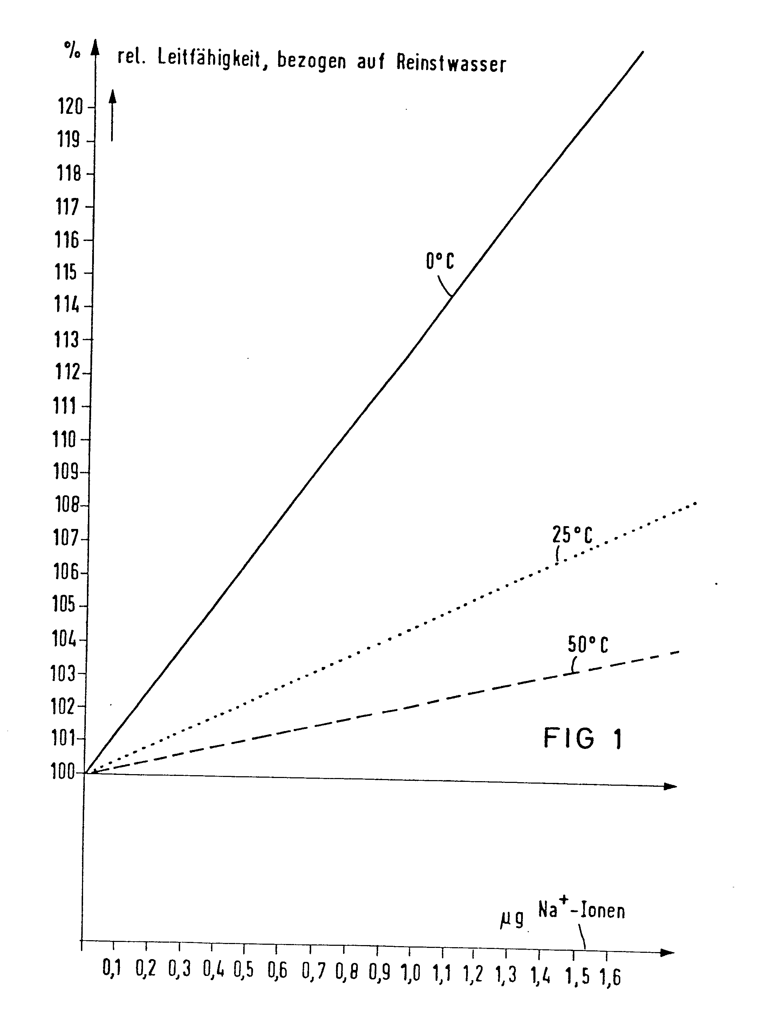

Zur Verdeutlichung der Vorteile sind in den Fig. 1 bis 3 Diagamme dargestellt, bei denen über dem Na+-Ionengehalt als Abszisse und der Leitfähigkeitsänderung, bezogen auf 100% Meßwert des Reinstwassers als Ordinate, Empfindlichkeitskurven mit der Temperatur als Parameter aufgetragen sind.

In Fig. 1 sind die Versuchsergebnisse bei der Verunreinigung von Reinstwasser durch Na+- und Cl−-Ionen dargestellt. Die Zugabe von NaCl zu dem Reinstwasser entspricht in der Praxis der sehr häufig vorkommenden Verunreinigung des Wassers durch Salze, insbesondere Steinsalz.

Man erkennt, daß mit sinkender Temperatur des zu untersuchenden Wassers die Empfindlichkeit erheblich erhöht wird. Die höchste Empfindlichkeit wird in der Nähe des Gefrierpunkts des Wassers erreicht.

Ein ähnliches Bild ergibt sich bei der Verunreinigung von Reinstwasser mit Säuren.

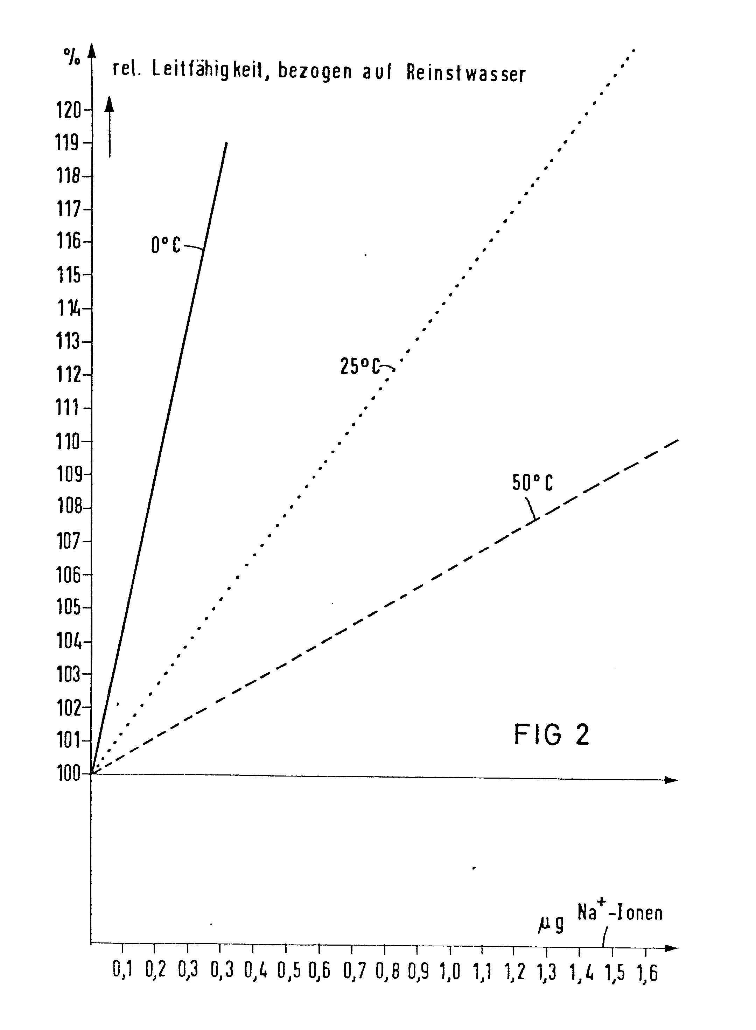

Bei den Versuchen, deren Ergebnisse in Fig. 2 dargestellt sind, wurde mit NaCl versetztes Reinstwasser durch einen Kationenaustauscher geleitet, gekühlt und dann die Leitfähigkeit gemessen. Dies würde in der Praxis einer äquimolaren Verunreinigung des Reinstwassers durch HCl entsprechen. Man erkennt auch hier die erhebliche Steigerung der relativen Empfindlichkeit bei Kühlung des untersuchten Wassers auf eine Temperatur in der Nähe seines Gefrierpunkts.

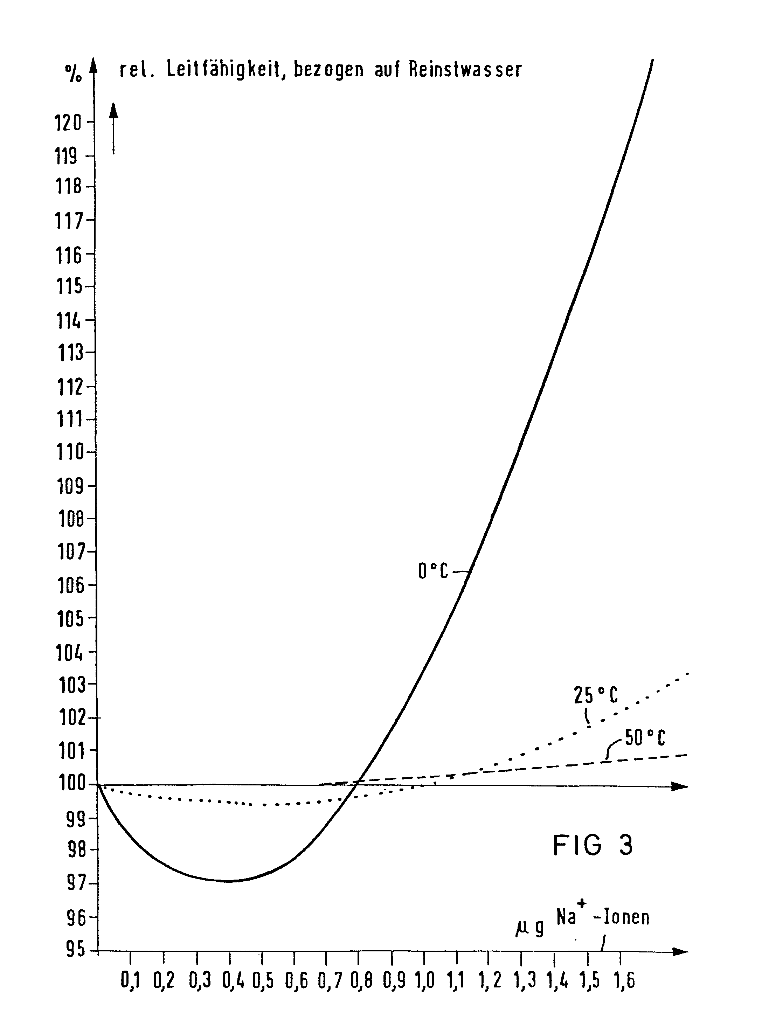

Wird der Fremdionengehalt des Reinstwassers durch Laugenspuren hervorgerufen, hier durch Zugabe von NaOH, durchlaufen die Meßkurven, wie aus Fig. 3 zu erkennen ist, eigenartigerweise einen Extremwert, der insbesondere bei der 0° C-Kurve deutlich unterhalb des Wertes für die Leitfähigkeit von Reinstwasser liegt. Meßwerte in diesem Bereich lassen zusätzlich mit Sicherheit darauf schließen, daß die Reaktion des Wassers alkalisch ist. Der pH-Wert liegt dann, auf 25° C bezogen, zwischen $pH = 7$ und $pH = 7,1$, wie es in den meisten Anwendungsfällen der Reinstwasseraufbereitung verlangt wird. Eine gleichzeitige Erfassung des Elektrolytgehaltes und der Alkalität im kontiuierlichen Betrieb ist mit anderen Methoden nicht oder nur mit großem Aufwand möglich.

Wird die Leitfähigkeit des Wassers vor und nach der Kühlung gemessen, kann aus den Meßwerten der Temperaturkoeffizient der Leifähigkeit ermittelt werden.

## Patentansprüche

1. Verfahren zur Überwachung von Reinstwasser auf Fremdionengehalt mit Hilfe einer Leitfähigkeitsmessung, dadurch gekennzeichnet, daß das Wasser vor der Messung auf eine mindestens annähernd konstante Temperatur zwischen 0° C und 5° C gekühlt wird.

2. Verfahren nach Anspruch 1, dadurch gekennzeichnet, daß das Wasser auf eine Temperatur in unmittelbarer Nähe seines Gefrierpunktes gekühlt wird.

## Claims

1. A method of monitoring highly pure water with respect to an alien ion content with the assistance of a conductivity measurement, characterised in that, prior to the measurement, the water is cooled to a temperature of between 0°C and 5°C which is at least approximately constant.

2. A method as claimed in Claim 1, characterised in that the water is cooled to a temperature in the immediate vicinity of its freezing point.

## Revendications

1. Procédé pour surveiller la teneur en ions étrangers de l'eau très pure au moyen d'une mesure de conductibilité, caractérisé en ce qu'il consiste à refroidir l'eau avant la mesure à une température au moins approximativement constante comprise entre 0°C et 5°C.

2. Procédé suivant la revendication 1, caractérisé en ce qu'il consiste à refroidir l'eau à une température au voisinage immédiat de son point de congélation.

FIG 1

FIG 2

FIG 3

rel. Leitfähigkeit, bezogen auf Reinstwasser

$\mu$ g Na$^+$ -Ionen